(19) **Europäisches Patentamt**
**European Patent Office**
**Office européen des brevets**

(11) **EP 2 313 777 B1**

(12) **EUROPEAN PATENT SPECIFICATION**

(45) Date of publication and mention
of the grant of the patent:
**02.07.2014 Bulletin 2014/27**

(51) Int Cl.:
*G01N 33/557* (2006.01)    *G01N 33/553* (2006.01)
*G01N 21/55* (2014.01)

(21) Application number: **09808459.3**

(22) Date of filing: **17.08.2009**

(86) International application number:
**PCT/SE2009/050936**

(87) International publication number:
**WO 2010/021586 (25.02.2010 Gazette 2010/08)**

(54) **A METHOD OF CHARACTERIZING ANTIBODIES**

VERFAHREN ZUR CHARACTERISIERUNG VON ANTIKÖRPERN

PROCÉDÉ DE CARACTÉRISATION D'ANTICORPS

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **22.08.2008 SE 0801824**

(43) Date of publication of application:
**27.04.2011 Bulletin 2011/17**

(73) Proprietor: **GE Healthcare Bio-Sciences AB
751 84 Uppsala (SE)**

(72) Inventors:
• **ANDERSSON, Karl
S-756 43 Uppsala (SE)**
• **KARLSSON, Robert
S-751 84 Uppsala (SE)**

(74) Representative: **Aldenbäck, Ulla Christina
GE Healthcare Bio-Sciences AB
Patent Department
Björkgatan 30
751 84 Uppsala (SE)**

(56) References cited:
**WO-A2-2004/046164      US-A1- 2005 175 999
US-A1- 2007 016 378**

• BRUDERER U ET AL: "Analyses of affinity distributions within polyclonal populations of antigen-specific antibodies - Evaluation of their accuracy in population detection using monoclonal antibodies", JOURNAL OF IMMUNOLOGICAL METHODS, ELSEVIER SCIENCE PUBLISHERS B.V.,AMSTERDAM, NL, vol. 151, no. 1-2, 6 July 1992 (1992-07-06), pages 157-164, XP023657584, ISSN: 0022-1759, DOI: 10.1016/0022-1759(92)90114-9 [retrieved on 1992-07-06]
• RILEY ET AL: "Stability of DNA/anti-DNA complexes. II. Salt lability and avidity.", THE JOURNAL OF IMMUNOLOGY, vol. 124, no. 1, 1 January 1980 (1980-01-01), pages 1-7, XP55003497, ISSN: 0022-1767
• HERRON J N ET AL: "Analysis of heterogeneous dissociation kinetics in polyclonal populations of rabbit anti-fluorescyl-IgG antibodies", MOLECULAR IMMUNOLOGY, PERGAMON, GB, vol. 20, no. 12, 1 December 1983 (1983-12-01), pages 1323-1332, XP023681149, ISSN: 0161-5890, DOI: 10.1016/0161-5890(83)90163-3 [retrieved on 1983-12-01]
• SEM D S ET AL: "Antibody affinities and relative titers in polyclonal populations: Surface plasmon resonance analysis of anti-DNA antibodies", ARCHIVES OF BIOCHEMISTRY AND BIOPHYSICS, ACADEMIC PRESS, US, vol. 372, no. 1, 1 December 1999 (1999-12-01), pages 62-68, XP002484631, ISSN: 0003-9861, DOI: 10.1006/ABBI.1999.1469

EP 2 313 777 B1

**(Cont. next page)**

- KRUSE V: "Dissociation rate constants and fractional binding of tracer estimated for three antibody populations in unstripped and stripped antiserum", SCANDINAVIAN JOURNAL OF CLINICAL & LABORATORY INVESTIGATION, INFORMA HEALTHCARE, LONDON, GB, vol. 39, no. 3, 1 May 1979 (1979-05-01), pages 215-221, XP009150662, ISSN: 0036-5513
- YUE D K ET AL: "The dissociation of insulin from human insulin antibodies", BIOCHIMICA ET BIOPHYSICA ACTA - GENERAL SUBJECTS, ELSEVIER SCIENCE PUBLISHERS, NL, vol. 444, no. 1, 24 August 1976 (1976-08-24), pages 231-239, XP025792166, ISSN: 0304-4165, DOI: 10.1016/0304-4165(76)90240-3 [retrieved on 1976-08-24]
- STECKBECK J.D. ET AL: 'Dynamic evolution of antibody populations in a rhesus macaque infected with attenuated simian immunodeficiency virus identified by surface plasmon resonance' J. MED. PRIMATOL. vol. 35, 2006, pages 248 - 260, XP003026093

**Description**

TECHNICAL FIELD OF THE INVENTION

The present invention relates to a method of characterizing antibodies, and more particularly characterizing a heterogeneous antibody population obtained in an immune response to a therapeutic drug or a vaccine.

BACKGROUND OF THE INVENTION

Immunogenicity is the ability to, or the degree to which a particular substance may provoke an immune response, such as the production of specific antibodies. The immune reaction may be desirable, as in the natural reaction of the immune defense system to infection, or in the provoked reaction to vaccines. The immune reaction may, however, also be unwanted, as when induced antibodies counteract the effect of administered drugs.

In recent years, immunogenicity has gained increasing focus in the context of immune reaction to biotherapeutics, often referred to as "biologics". Biotherapeutics or biologics are large-molecule drugs or medicines based on proteins, peptides and antibodies that primarily come from molecular biology developments. Usually, the terms also includes vaccines.

As more biomolecules, such as natural or engineered inhibitors, effectors or antibodies are used as therapeutics, issues related to the immunogenicity of the molecules become more important.

Both the occurrence and the effects of an immune response to biologics vary widely. Administration of a biotherapeutic drug does not always elicit an immune response in the patient, and the same drug may cause a response in some patients but not in others. On the other hand, many drugs elicit an immune response without affecting the efficacy of the treatment, so that the detected production of antibodies does not necessarily indicate that the immune response will be clinically relevant.

Undesired effects of the immune response to biologics include reduced or complete neutralization of the effect of the desired effect of therapy, formation of antibody-drug complexes which may effect the pharmacokinetic properties of the agent, as well as undesirable side-effects, which sometimes may be analogous to auto-immune conditions.

For a biotherapeutic drug, the goal is usually to avoid or minimize the immune response, at least in terms of clinically relevant antibodies, and characterization of the immunogenic properties of the drug is therefore a critical issue in the drug development.

Development of vaccines also rely on characterization of immunogenicity, but from a different perspective than biotherapeutic development. Vaccines are designed to elicit a long-lived immune defense reaction against the pathogen, and low levels of antibody production are therefore generally uninteresting. Any clinical side-effects of the antibody need to be studied, but the primary characteristic of the antibody is that it should neutralize the effect of the pathogen.

Antibodies elicited in response to a vaccine or a biotherapeutic, such as a protein drug, represent a broad spectrum of classes and affinities. The antibody response to an immunogen generally also changes over time in a single individual, as the mixture of antibody classes and affinities changes. Physical and clinical effects of the immune response may change as the antibody population changes. Immunogenicity in the context of biotherapeutic treatments is thus a complex issue where the behaviour and effects of the immune response may vary both from case to case and over time.

Analytical sensor systems that can monitor molecular interactions in real time are gaining increasing interest. These systems are often based on optical biosensors and usually referred to as interaction analysis sensors or biospecific interaction analysis sensors. A representative such biosensor system is the BIACORE® instrumentation sold by GE Healthcare (Uppsala, Sweden) which uses surface plasmon resonance (SPR) for detecting interactions between molecules in a sample and molecular structures immobilized on a sensor surface. With the BIACORE® systems it is possible to determine in real time without the use of labeling not only the presence and concentration of a particular molecule in a sample, but also additional interaction parameters such as, for instance, the association rate and dissociation rate constants for the molecular interaction.

By immobilizing a therapeutic drug to the sensing surface and measuring the response when the surface is contacted with antibody-containing samples, the presence of antibodies specific for the drugs can be screened. The response level obtained reflects the amount of antibody in the sample. By utilizing an acidification/neutralization procedure as disclosed in WO 08/033073 A1, also antibodies complexed with drug in the samples can be detected.

Steckbeck J.D. et al., J. Med. Primatolog., vol. 35, 2006, pages 248-260 describes a method to study the antibody binding properties of polyclonal serum antibody responses generated in rhesus macaques infected or vaccinated with attenuated Simian immunodeficiency virus (SIV). The dissociation behavior of the antibody populations were analyzed using SPR where the serum antibodies are captured on the surface of the sensor chip and varying concentrations of the antigen were passed sequentially over the sensor chip. By measuring binding kinetics, two antibody subpopulations are identified in the serum samples: one "unstable" antibody population with fast association/dissociation rates and a second "stable" antibody population with slower association/dissociation rates. The kinetic rates were evaluated using the Langmuir binding model.

US2007/016378A1 demonstrates how to use and modify the Langmuir binding model to measure molecular interaction between a surface-bound ligand and an analyte using SPR. The model assumes that the analyte is both monovalent and homogeneous, that the ligand is homogeneous, and that all binding events are independent.

Bruderer U. et al., Journal of Immunological Methods, vol 151, no 1-2, July 1992, pages 157-164 assesses ELISA-based detection of affinity distributions within polyclonal populations of *P.aeruginosa* exotoxin A-specific serum antibodies by analyzing defined probes composed of monoclonal antibodies. Results indicated that the detectability of antibody populations depends on the antigen concentrations in the solid phase and on the affinity distribution of the probe to be analyzed. In wells coated with high antigen concentrations, antibody titers reflected antibody concentration, whereas at low antigen concentrations antibody titers primarily reflected antibody affinities. Based on the measurement, different subpopulations of polyclonal antibodies were identified. It is proposed the methodology could be applied for assessment of vaccine preparations.

Riley et al., Journal of Immunology, vol 124, no 1, January 1980, pages 1-7 studies the kinetics of dissociation of antibody-radiolabeled dsDNA via three independent radioimmunoassays. Either excess unlabeled DNA or high salt concentrations were employed to induce complex dissociation. Results that typical SLE (Systemic Lupus Erythematosus) sera contain at least two distinct populations of anti-dsDNA antibodies. One population is of rather high avidity and dissociates slowly in the presence of excess DNA or high salt. The other population is of considerably lower avidity and is dissociated more rapidly under these conditions. The author proposes to employ the method for analysis of the pathogenic bases of SLE.

In Herron J.N. et al., Molecular Immunology, Pergamon, GB, vol. 20, no 12, December 1993, pages 1323-1332 hapten dissociation kinetics were used to characterize the expression of hyperimmune anti-fluorescyl-IgG antibodies within a population of rabbits. Antibody preparations exhibited at least two discrete dissociative components, which were characterized by different dissociation rates and dissociative lifetimes. These findings implied that affinity maturation of the anti-fluorescyl IgG antibody response involved the generation of discrete high-affinity subpopulations of antibodies rather than a continuous spectrum of high-affinity antibodies.

Sem D. S. et al., Archives of Biochemistry and Biophysics, Academic press, US, vol. 373, no 1, December 1999, pages 62-68 present a methodology for the measurement and interpretation of apparent dissociation constants, including equations, for polyclonal populations of antibodies. SPR was used to determine Kd for the binding of anti-DNA antibodies to trace amounts of DNA antigen on a chip. The apparent Kd is a weighted average of all the Kd for the clonally related subpopulations within the polyclonal pool, where each weighting factor is the relative titer (fractional presence) of the subpopulation. Implications of changes in the antibody affinity distribution within the population are discussed (abstract, materials and methods).

Kruse V., Scandinavian Journal of Clinical and Laboratory Investigation, Informa Healthcare, London, GB, vol 39, no 3, May 1979, pages 215-221 estimates dissociations rate constants of a high affinity thyroxine antiserum. Dissociation curves were fitted by computer to an equation with a sum of two or three exponentials. The analysis of the curves gave estimates of the dissociation rate constants for fast, medium and slowly dissociating antibody populations, and also estimates of the size of the fraction of tracer bound to each of the three populations, and also estimates of the size of the fraction of tracer bound to each of the three populations of antibodies.

Yue D.K. et al" Biochimica et Biophysica Acta- General subjects, Elsevivier publishers, NL, vol 444, no 1. August 1976, pages 231-239 analyses the dissociation of insulin from human insulin analogues. A slow and fast dissociating antibody component were identified in all studies. There were shown to correspond, respectively, to the high and low affinity antibody components of equilibrium binding studies. The author discuss how therapeutically injected insulin bound to antibodies impacts therapy. Since, as mentioned above, the antibody population obtained in response to a vaccine or a biotherapeutic, may represent a broad spectrum of affinities and classes, the binding and dissociation of antibody from the sensor surface is complex. It is readily seen that characterizing such an antibody population is a tedious and laborious task. There is therefore a need for sensor-based methods which permit easy characterization and comparison of immune response-derived antibody populations.

SUMMARY OF THE INVENTION

The above and other objects and advantages are provided by a method which characterizes the antibodies in terms of the stability of binding to a drug immobilized to a sensor surface, and more particularly by assessing the immune complex stability by studying the dissociation of bound antibodies and classifying the antibody population in terms of predominance of rapidly or slowly dissociating antibodies.

In one aspect, the present invention provides a method of characterizing a population of antibodies to an antigen, which comprises providing SPR sensor surfaces or discrete areas of a SPR sensor surface having different domains of the antigen immobilized thereto in different densities, contacting the sensor surface or surfaces with a sample containing the antibody population to bind antibodies to the surface, detecting dissociation of antibodies from the sensor surface during a dissociation phase when sample no longer contacts the surface, wherein the dissociation behaviour of the antibody population is characterized by analyzing a first subpopulation and a second subpopulation of said antibody population, and wherein antibodies in said first subpopulation form a more stable complex with the immobilized antigen than antibodies in said second subpopulation, and wherein the method comprises fitting dissociation phase detection data to a kinetic model which describes simultaneous dissociation from two independent monovalent sites, one with a faster dissociation phase and one with a slower dissociation phase.

The antigen may be a therapeutic drug, preferably a biotherapeutic, or a vaccine.

In another aspect, the present invention provides a method of determining the immunogenicity of drug, which comprises monitoring the appearance of anti-drug antibodies in a patient over time by the method of the first-mentioned aspect, and determining any shift in proportions between more stable and less stable antibodies.

A more complete understanding of the present invention, as well as further features and advantages thereof, will be obtained by reference to the following detailed description and drawings.

BRIF DESCRIPTION OF THE DRAWINGS

Figure 1 is a schematic side view of a biosensor system based on SPR.
Figure 2 is a representative sensorgram showing detector response versus time for the interaction between an analyte and an immobilized binder for the analyte.

DETAILED DESCRIPTION OF THE INVENTION

Unless defined otherwise, all technical and scientific terms used herein have the same meaning as commonly understood by a person skilled in the art related to this invention. Also, the singular forms "a", "an", and "the" are meant to include plural reference unless it is stated otherwise.

As mentioned above, the invention relates to the characterization of antibody populations produced in response to an immune reaction provoked by an immunogen. In brief, the antibody population is characterized in terms of the stability of the antibody-antigen complex formed when a sample containing a heterogeneous antibody population is contacted with a sensor surface with immobilized antigen. More particularly, the stability of the antibody-antigen complex is determined by studying the dissociation behaviour of bound antibodies at the sensor surface.

The antibody population may represent a broad spectrum of affinities within and between different antibody classes, and the dissociation behaviour of antibody from the sensor surface is correspondingly complex. Attempting to describe the dissociation behaviour of such a population in terms of a single interaction does not give satisfactory results, partly because of the heterogenicity of the antibody population, and partly because antibodies are normally bi- or multivalent. Also, it is not practicable to resolve the behaviour into components that represent individual antibody species.

According to the invention, as a compromise, the dissociation behaviour is considered to be composed of the averaged behaviours of two antibody subpopulations, one forming a less stable complex with antigen (showing rapid dissociation behaviour) and one subpopulation forming a more stable complex with antigen (showing a slower dissociation behaviour). Notably, while this does not imply that there are two distinct populations of antibodies in the sample, this approach is an empirical compromise that describes the behaviour satisfactorily and helps to classify the immune response in terms of predominance of rapidly or slowly dissociating antibody subpopulations. The antibody population may then conveniently be characterized in terms of the subpopulations or fractions of rapidly and slowly, respectively, dissociating antibodies.

The sensor used in the present invention is typically a biosensor. Biosensors are usually based on label-free techniques, detecting a change in a property of a sensor surface, such as e.g. mass, refractive index, or thickness for the immobilised layer, but there are also sensors relying on some kind of labelling. Typical sensor detection techniques include, but are not limited to, mass detection methods, such as optical, thermo-optical and piezoelectric or acoustic wave methods (including e.g. surface acoustic wave (SAW) and quartz crystal microbalance (QCM) methods), and electrochemical methods, such as potentiometric, conductometric, amperometric and capacitance/impedance methods. With regard to optical detection methods, representative methods include those that detect mass surface concentration, such as reflection-optical methods, including both external and internal reflection methods, which are angle, wavelength, polarization, or phase resolved, for example evanescent wave ellipsometry and evanescent wave spectroscopy (EWS, or Internal Reflection Spectroscopy), both of which may include evanescent field enhancement via surface plasmon resonance (SPR), Brewster angle refractometry, critical angle refractometry, frustrated total reflection (FTR), scattered total internal reflection (STIR) (which may include scatter enhancing labels), optical wave guide sensors; external reflection imaging, evanescent wave-based imaging such as critical angle resolved imaging, Brewster angle resolved imaging, SPR-angle resolved imaging, and the like. Further, photometric and imaging/microscopy methods, "per se" or combined with reflection methods, based on for example surface enhanced Raman spectroscopy (SERS), surface enhanced resonance Raman spectroscopy (SERRS), evanescent wave fluorescence (TIRF) and phosphorescence may be mentioned, as well as waveguide interferometers, waveguide leaky mode spectroscopy, reflective interference spectroscopy (RIfS), transmission interferometry, holographic spectroscopy, and atomic force microscopy (AFR).

Commercially available biosensors include the afore-mentioned BIACORE® systems, manufactured and marketed by GE Healthcare, Uppsala, Sweden, and the PROTEON™ XPR36 system, manufactured and marketed by Bio-Rad Laboratories Inc., CA, USA), which are both based on surface plasmon resonance (SPR) and permit monitoring of surface binding interactions in real time between a bound ligand and an analyte of interest. In this context, "ligand" is a molecule that has a known or unknown affinity for a given analyte and includes any capturing or catching agent immobilized on the surface, whereas "analyte" includes any specific binding partner thereto.

While in the detailed description and Examples that follow, the present invention is illustrated in the context of SPR spectroscopy, and more particularly the BIACORE® systems, it is to be understood that the present invention is not limited to this detection method. Rather, any affinity-based detection method where an analyte binds to a ligand immobilised on a sensing surface may be employed, provided that a change at the sensing surface can be measured which is quantitatively indicative of binding of the analyte to the immobilised ligand thereon.

The phenomenon of SPR is well known, suffice it to say that SPR arises when light is reflected under certain conditions at the interface between two media of different refractive indices, and the interface is coated by a metal film, typically silver or gold. In the BIACORE® instruments, the media are the sample and the glass of a sensor chip which is contacted with the sample by a micro fluidic flow system. The metal film is a thin layer of gold on the chip surface. SPR causes a reduction in the intensity of the reflected light at a specific angle of reflection. This angle of minimum reflected light intensity - the resonance angle - varies with the refractive index close to the surface on the side opposite from the reflected light, in the BIACORE® system the sample side. A change of refractive index close to the surface, resulting from e.g. molecular binding thereto, causes a shift in resonance angle and this shift is measured as a response signal.

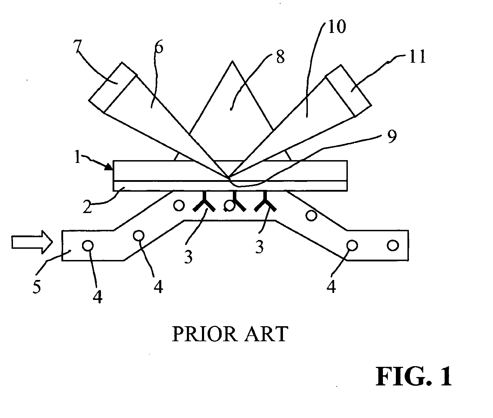

A schematic illustration of the BIACORE® system is shown in Fig. 1. Sensor chip 1 has a gold film 2 supporting capturing molecules (ligands) 3, e.g. antibodies, exposed to a sample flow with analytes 4, e.g. an antigen, through a flow channel 5. Monochromatic p-polarised light 6 from a light source 7 (LED) is coupled by a prism 8 to the glass/metal interface 9 where the light is totally reflected. The intensity of the reflected light beam 10 is detected by an optical detection unit 11 (photodetector array).

A detailed discussion of the technical aspects of the BIACORE® instruments and the phenomenon of SPR may be found in U.S. Patent No. 5,313,264. More detailed information on matrix coatings for biosensor sensing surfaces is given in, for example, U.S. Patent Nos. 5,242,828 and 5,436,161. In addition, a detailed discussion of the technical aspects of the biosensor chips used in connection with the BIACORE® instruments may be found in U.S. Patent No. 5,492,840.

When molecules in the sample bind to the capturing molecules on the sensor chip surface, the concentration, and therefore the refractive index at the surface changes and an SPR response is detected. Plotting the response against time during the course of an interaction will provide a quantitative measure of the progress of the interaction. Such a plot, or kinetic or binding curve (binding isotherm), is usually called a sensorgram, also sometimes referred to in the art as "affinity trace" or "affinogram". In the BIACORE® systems, the SPR response values are expressed in resonance units (RU). One RU represents a change of 0.0001° in the angle of minimum reflected light intensity, which for most proteins and other biomolecules correspond to a change in concentration of about 1 pg/mm$^2$ on the sensor surface. As sample containing an analyte contacts the sensor surface, the capturing molecule (ligand) bound to the sensor surface interacts with the analyte in a step referred to as "association." This step is indicated on the sensorgram by an increase in RU as the sample is initially brought into contact with the sensor surface. Conversely, "dissociation" normally occurs when the sample flow is replaced by, for example, a buffer flow. This step is indicated on the sensorgram by a drop in RU over time as analyte dissociates from the surface-bound ligand.

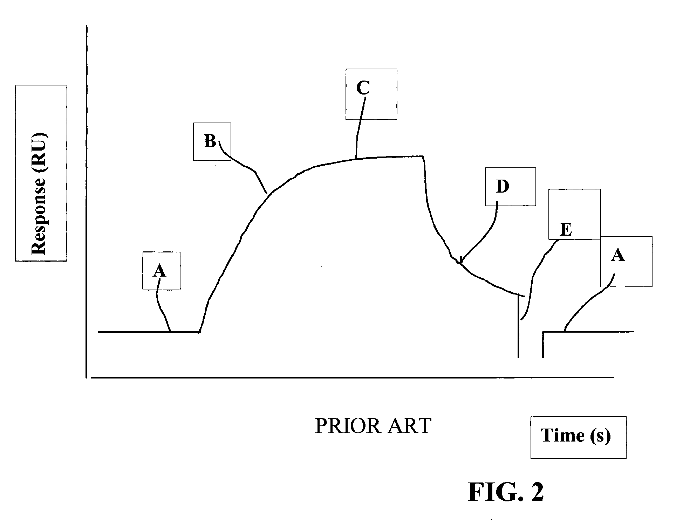

A representative sensorgram (binding curve) for a reversible interaction at the sensor chip surface is presented in Fig. 2, the sensing surface having an immobilised capturing molecule, or ligand, for example an antibody, interacting with a binding partner therefor, or analyte, in a sample. The binding curves produced by biosensor systems based on other detection principles mentioned above will have a similar appearance. The vertical axis (y-axis) indicates the response (here in resonance units, RU) and the horizontal axis (x-axis) indicates the time (here in seconds). Initially, buffer is passed over the sensing surface giving the baseline response $\underline{A}$ in the sensorgram. During sample injection, an increase in signal is observed due to binding of the analyte. This part $\underline{B}$ of the binding curve is usually referred to as the "association phase". Eventually, a steady state or equilibrium condition is reached at or near the end of the association phase where the resonance signal plateaus at $\underline{C}$ (this state may, however, not always be achieved). At the end of sample injection, the sample is replaced with a continuous flow of buffer and a decrease in signal reflects the dissociation, or release, of analyte from the surface. This part $\underline{D}$ of the binding curve is usually referred to as the "dissociation phase". The analysis is ended by a regeneration step where a solution capable of removing bound analyte from the surface, while (ideally) maintaining the activity of the ligand, is injected over the sensor surface. This is indicated in part $\underline{E}$ of the sensorgram. Injection of buffer restores the baseline $\underline{A}$ and the surface is now ready for a new analysis.

From the profiles of the association and dissociation phases $\underline{B}$ and $\underline{D}$, respectively, information regarding the binding and dissociation kinetics is obtained, and the height of the resonance signal at $\underline{C}$ represents affinity (the response resulting from an interaction being related to the change in mass concentration on the surface).

The rate of dissociation in the dissociation phase can be expressed as:

$$\frac{dR}{dt} = -k_d R$$

and in integrated form:

$$R = R_0 \cdot e^{-k_d t}$$

where R is the response at time $\underline{t}$ in resonance units (RU), $R_0$ is the response at the beginning of the dissociation phase (when the buffer wash of the surface starts), and $k_d$ is the dissociation rate constant.

And now back to the present invention. As mentioned the dissociation behaviour is considered to be composed of the averaged behaviours of two antibody subpopulations originating from one and the same antibody population, one with more rapid dissociation and one with slower dissociation. To this end, dissociation kinetics are typically determined by fitting the dissociation phase of the sensorgrams to an equation which describes simultaneous dissociation from two independent monovalent sites on the antigen:

$$R = R_1 * e^{-k_{d1}(t-t_0)} + R_2 * e^{-k_{d2}(t-t_0)}$$

where R is the detection response time at time t, $R_1$ and $R_2$ are the response contributions from the two sites at the beginning of the dissociation at time to, and $k_{d1}$ and $k_{d2}$ are the dissociation rate constants for the two sites.

It is to be noted that this equation is based on simple monovalent dissociation kinetics at the two sites. Antibodies are usually bi- or multivalent, which complicates the dissociation behaviour further. However, while the two-site monovalent model provides a sufficiently good empirical description of the observed dissociation in most cases, it should be realized that the dissociation characteristics reported by the evaluation are empirical and do not represent formal kinetic parameters.

For presentation and evaluation of the results, the apparent dissociation rate constants determined by the fitting procedure are usually converted to half-lives, $t_{1/2}$, i.e. the time required for the amount of complex to be reduced by 50%:

$$t_{1/2} = \frac{\ln 2}{k_d}$$

If the dissociation behaviour describes a true exponential curve and can therefore in principle be analyzed with a single site model, the two-site fitting procedure will typically return either one population with a very low fraction and a non-significant value for $t_{1/2}$ or two populations with closely similar values for $t_{1/2}$ and an unpredictable division of the response between the populations. Also, if either one fraction is calculated as less than, say, 1% or the values for $t_{1/2}$ in the two populations differ by less than, say, 10%, the evaluation will typically assign the whole antibody population to the slow fraction and report the fast fraction as 0%.

Conveniently, the results from the fitting are described by four parameters.

- Fraction fast

- tt$_{1/2}$ fast

- Fraction slow

- $t_{1/2}$ slow

It is to be noted that the terms fast (rapid) and slow do not refer to any absolute values for $t_{1/2}$, but are applied individually to the fitted results for each separate sample.

With the above-mentioned BIACORE® system, for example, the dissociation data selected for the fitting should be selected shortly after the end of the sample contact with the sensor surface to minimize interference from bulk refractive index contributions in the sample which may otherwise be interpreted as rapid dissociation events and contribute to the reported fast population of antibodies.

As mentioned above, antibodies elicited in response to a vaccine or to a biotherapeutic usually represent broad spectrum of classes and affinities. Not only may the antibodies have different kinetic properties, but they may also compete for the same epitope and/or there may be avidity effects.

Preferably, the sample is therefore contacted with sensor surfaces or surface areas having different antigen domains (rather than or in addition to intact antigen) immobilized thereto. By subdividing the antigen into parts in this way, it will thus be possible to detect site specific antibodies. The different antigen parts, which may be produced by recombinant DNA technology as is well known in the art, may be immobilized on separate sensor surfaces or on separate areas or spots of a linear or two-dimensional array type sensor surface.

To be able to compensate for avidity effects that may influence the apparent affinities of the antibodies, the sample is preferably contacted with sensor surfaces or surface areas varying densities of the immobilized antigen or antigens.

The above-mentioned four parameters fraction fast, $t_{1/2}$ fast, fraction slow and $t_{1/2}$ slow are the pattern obtained from the different surfaces or surface areas are then used to describe and rank the total polyclonal antibody response.

In case of therapeutic drugs, since an immune response may cause side effects or neutralize the drug, it is of interest to monitor individual patients to determine any shift over time in apparent stability of antibody binding and any shift in the proportions between more stable and less stable antibodies.

Also, in the case of therapeutic drugs, the present antibody characterization method may be used to group responders and find parameters that can be used to predict relevance of different response patterns for a successful therapy.

In vaccine testing, the antibody characterization method may be used to test that antibodies are expressed as expected and the patterns obtained can be indicative of whether a useful immune response has been obtained or not.

In the following Examples, various aspects of the present invention are disclosed more specifically for purposes of illustration and not limitation.

EXAMPLES

Instrumentation

A BIACORE® T100 (GE Healthcare, Uppsala, Sweden) was used. This instrument, which is based on surface plasmon resonance (SPR) detection at a gold surface on a sensor chip, uses a micro-fluidic system (integrated micro-fluidic cartridge - IFC) for passing samples and running buffer through four individually detected flow cells, designated Fc1 to Fc 4, one by one or in series. The IFC is pressed into contact with the sensor chip by a docking mechanism within the BIACORE® T 100 instrument.

As sensor chip was used Series CM5 (GE Healthcare, Uppsala, Sweden) which has a gold-coated (about 50 nm) surface with a covalently linked hydrogel matrix (about 100 nm) of carboxymethyl-modified dextran polymer.

The output from the instrument is a "sensorgram" which is a plot of detector response (measured in "resonance units", RU) as a function of time. An increase of 1000 RU corresponds to an increase of mass on the sensor surface of approximately 1 ng/mm$^2$.

**Example 1**

**Binding stability analysis on surface with low ligand density**

A model system consisting of beta-2-microglobulin as ligand and a polyclonal anti-beta-2-microglobulin antibody as analyte was used. Beta-2-microglobulin was immobilized on sensor chip CM5 using Amine Coupling Kit (GE Healthcare, Uppsala, Sweden) according to the manufacturer's instructions. The immobilization was performed in the presence of ethanolamine to obtain a sufficiently low ligand density.

The analysis was performed in the BIACORE® T100 instrument including "Immunogenicity Package" software.

Five different concentrations of the polyclonal antibody diluted in HBS-EP+ were used: 5, 10, 25, 50 and 100 μg/ml. Two different injection times were tested, 60 and 90 seconds, respectively, while the dissociation time was held constant to 600 seconds. The analysis was performed with the instruments's "Method Builder" support and comprised four assay steps.

Assay Step 1 5-100 $\mu$g/ml, injection for 60 s
Assay Step 2 5-100 $\mu$g/ml, injection for 90 s
Assay Step 3 5-100 $\mu$g/ml, injection for 60 s
Assay Step 4 5-100 $\mu$g/ml, injection for 90 s

Four Start Up cycles were run before analysis and between each Assay Step a control sample with buffer was run. Only flow cells Fc3 and Fc4 were used in the analysis. Immobilization in Fc4 and Fc3 were used as reference. The flow rate was 30 $\mu$l/min.

The analysis was evaluated in BIACORE® T100 Evaluation Software 2.0. The obtained values that were evaluated are Fast Fraction (%), $t_{1/2}$ Fast (s), $t_{1/2}$ Slow (s) and Chi$^2$. The results are presented in Table 1 below.

Table 1

| Cycle# | Conc (µg/ml) | Inject. Time (s) | Fast Fraction (%) | Slow Fraction (%) | t½ Fast (s) | t½ Slow (s) | RelResp Tot (RU) | RelResp Fast (RU) | RelResp Slow (RU) | Chi² (RU²) |
|---|---|---|---|---|---|---|---|---|---|---|
| 6 | 5 | 60 | 60 | 40 | 42 | 5,00E+02 | 1,3 | 0,8 | 0,5 | 0,003 |
| 7 | 10 | 60 | 26 | 74 | 59 | 2,60E+03 | 2,9 | 0,7 | 2,1 | 0,0028 |
| 8 | 25 | 60 | 24 | 76 | 87 | 4,60E+03 | 6,1 | 1,5 | 4,7 | 0,0031 |
| 9 | 50 | 60 | 19 | 81 | 72 | 3,10E+03 | 9,6 | 1,8 | 7,8 | 0,0031 |
| 10 | 100 | 60 | 18 | 82 | 80 | 3,10E+03 | 13,9 | 2,5 | 11,4 | 0,0035 |
| 12 | 5 | 90 | 52 | 48 | 94 | 5,80E+03 | 1,7 | 0,9 | 0,8 | 0,0029 |
| 13 | 10 | 90 | 20 | 80 | 54 | 2,80E+03 | 3,9 | 0,8 | 3,1 | 0,0034 |
| 14 | 25 | 90 | 19 | 81 | 83 | 3,90E+03 | 7,7 | 1,5 | 6,2 | 0,0028 |
| 15 | 50 | 90 | 16 | 84 | 76 | 3,00E+03 | 11,6 | 1,8 | 9,7 | 0,0031 |
| 16 | 100 | 90 | 15 | 85 | 70 | 2,70E+03 | 16,1 | 2,4 | 13,8 | 0,0034 |
| 18 | 5 | 60 | 55 | 45 | 41 | 5,00E+02 | 1,3 | 0,7 | 0,6 | 0,0027 |
| 19 | 10 | 60 | 25 | 75 | 62 | 2,80E+03 | 2,9 | 0,7 | 2,1 | 0,0035 |
| 20 | 25 | 60 | 21 | 79 | 68 | 3,60E+03 | 6 | 1,2 | 4,7 | 0,003 |
| 21 | 50 | 60 | 17 | 83 | 72 | 2,90E+03 | 9,3 | 1,6 | 7,7 | 0,0029 |
| 22 | 100 | 60 | 17 | 83 | 60 | 2,50E+03 | 13,9 | 2,3 | 11,6 | 0,0033 |
| 24 | 5 | 90 | 46 | 54 | 67 | 2,70E+03 | 1,6 | 0,8 | 0,9 | 0,0032 |
| 25 | 10 | 90 | 18 | 82 | 55 | 2,40E+03 | 3,8 | 0,7 | 3,1 | 0,0027 |
| 26 | 25 | 90 | 15 | 85 | 48 | 2,40E+03 | 7,7 | 1,1 | 6,6 | 0,0028 |
| 27 | 50 | 90 | 16 | 84 | 79 | 3,10E+03 | 11,4 | 1,8 | 9,6 | 0,003 |
| 28 | 100 | 90 | 15 | 85 | 76 | 2,90E+03 | 16 | 2,4 | 13,6 | 0,0029 |

As appears from Table 1, it is mainly the lowest concentration, 5 $\mu$g/ml, that causes to the dispersion of the obtained values, which, however, be due to the low detector response obtained for this concentration. If the 5 $\mu$g/ml concentration is disregarded, the result would be 15-26% for Fast Fraction (%), 48-87 s for Fast (s), $t_{1/2}$Fast (s), and 2400-4600 s for $t_{1/2}$ Slow (s).

It is to be understood that the invention is not limited to the particular embodiments of the invention described above, but the scope of the invention will be established by the appended claims

Claims

1.   A method of characterizing a population of antibodies to an antigen, which comprises providing SPR (surface plasmon resonance) sensor surfaces or discrete areas of a SPR sensor surface having different domains of the antigen immobilized thereto in different densities, contacting the sensor surface or surfaces with a sample containing the antibody population to bind antibodies to the surface, detecting dissociation of antibodies from the sensor surface during a dissociation phase when sample no longer contacts the surface, wherein the dissociation behaviour of the antibody population is **characterized by** analyzing a first subpopulation and a second subpopulation of said antibody population, and wherein antibodies in said first subpopulation form a more stable complex with the immobilized antigen than antibodies in said second subpopulation, and wherein the method comprises fitting dissociation phase detection data to a kinetic model which describes simultaneous dissociation from two independent monovalent sites, one with a faster dissociation phase, and one with a slower dissociation phase.

2. The method according to claim 1, wherein the kinetic model is a two-component equation:

$$R = R_1 * e^{-k_{d1}(t-t_0)} + R_2 * e^{-k_{d2}(t-t_0)}$$

where R is the detection response time at time t, $R_1$ and $R_2$ are the response contributions from the sites with the faster and slower dissociation phases, respectively, at the beginning of the dissociation at time to, and $k_{d1}$ and $k_{d2}$ are the dissociation rate constants for the sites with the faster and slower dissociation phases, respectively.

3. The method according to claim 2, wherein the dissociation rate constants determined by the fitting are converted to half-lives ($t_{1/2}$) by the equation:

$$t_{1/2} = \frac{\ln 2}{k_d}$$

where $t_{1/2}$ is the time required for the amount of antibody-antigen complex to be reduced to 50% during dissociation.

4. The method according to claim 3, wherein the results of the fitting are presented by four parameters: fraction fast, $t_{1/2}$ fast, fraction slow, $t_{1/2}$ slow.

5. The method according to any one of claims 1 to 4, wherein the antigen is a drug.

6. The method according to any one of claims 1 to 4, wherein the antigen is a vaccine.

7. A method of determining the immunogenicity of drug, which comprises monitoring the appearance of anti-drug antibodies in a patient over time by the method according to any one of claims 1 to 4, and determining any shift in proportions between more stable and less stable antibodies.

**Patentansprüche**

1. Verfahren zum Charakterisieren einer Population von Antikörpern gegen ein Antigen, das ein Bereitstellen von SPR-(Surface Plasmon Resonance) Sensorflächen oder einzelner Bereiche einer SPR-Sensorfläche, an welchen unterschiedliche Domänen des Antigens in unterschiedlichen Dichten immobilisiert sind, ein Kontaktieren der Sensorfläche oder -flächen mit einer Probe, die die Antikörperpopulation enthält, um Antikörper an die Fläche zu binden, ein Erfassen einer Trennung von Antikörpern von der Sensorfläche während einer Trennungsphase, wenn die Probe nicht mehr länger mit der Fläche in Kontakt ist, umfasst, wobei das Trennungsverhalten der Antikörperpopulation durch Analysieren einer ersten Subpopulation und einer zweiten Subpopulation der Antikörperpopulation charakterisiert ist und wobei Antikörper in der ersten Subpopulation einen stabileren Komplex mit dem immobilisierten Antigen bilden als Antikörper in der zweiten Subpopulation und wobei das Verfahren ein Anpassen von Trennungsphasenerfassungsdaten an ein kinetisches Modell umfasst, das eine gleichzeitige Trennung von zwei unabhängigen einwertigen Stellen beschreibt, einer mit einer schnelleren Trennungsphase und einer mit einer langsameren Trennungsphase.

2. Verfahren nach Anspruch 1, wobei das kinetische Modell eine Zweikomponenten-Gleichung ist:

$$R = R_1 * e^{-k_{d1}(t-t_0)} + R_2 * e^{-k_{d2}(t-t_0)}$$

wobei R die Erfassungsreaktionszeit zum Zeitpunkt t ist, $R_1$ und $R_2$ die Reaktionsbeiträge von den Stellen mit der schnelleren bzw. langsameren Trennungsphase zu Beginn der Trennung zum Zeitpunkt $t_0$ sind und $k_{d1}$ und $k_{d2}$ die Trennungsratenkonstanten für die Stellen mit der schnelleren bzw. langsameren Trennungsphase sind.

3. Verfahren nach Anspruch 2, wobei die Trennungsratenkonstanten, die durch die Anpassung bestimmt werden, durch die Gleichung:

$$t_{1/2} = \frac{ln2}{k_d}$$

zu Halbwertszeiten ($t_{1/2}$) umgewandelt werden, wobei $t_{1/2}$ die Zeit ist, die notwendig ist, damit ein Antikörper-Antigen-Komplex während der Trennung auf 50% reduziert wird.

4. Verfahren nach Anspruch 3, wobei die Ergebnisse der Anpassung durch vier Parameter angegeben sind: Fraktion schnell, $t_{1/2}$ schnell, Fraktion langsam, $t_{1/2}$ langsam.

5. Verfahren nach einem der Ansprüche 1 bis 4, wobei das Antigen ein Arzneimittel ist.

6. Verfahren nach einem der Ansprüche 1 bis 4, wobei das Antigen ein Impfstoff ist.

7. Verfahren zum Bestimmen der Immunogenität eines Arzneimittels, das ein Überwachen des Erscheinens von Anti-Arzneimittel-Antikörpern in einem Patienten im Laufe der Zeit durch das Verfahren nach einem der Ansprüche 1 bis 4 und ein Bestimmen einer Verschiebung in Anteilen zwischen stabileren und weniger stabilen Antikörpern umfasst.

## Revendications

1. Une méthode de caractérisation d'une population d'anticorps à un antigène, qui comprend la fourniture de surfaces de capteur SPR (résonance plasmonique de surface) ou de zones discrètes d'une surface de capteur SPR ayant des domaines différents de l'antigène immobilisé dans ces dernières dans des densités différentes, la mise en contact de la surface ou des surfaces de capteur avec un échantillon contenant la population d'anticorps pour lier des anticorps à la surface, la détection d'une dissociation d'anticorps depuis la surface de capteur pendant une phase de dissociation quand l'échantillon n'entre plus en contact avec la surface, dans laquelle le comportement de dissociation de la population d'anticorps est caractérisé en analysant une première sous-population et une seconde sous-population de ladite population d'anticorps, et dans laquelle des anticorps dans ladite première sous-population forment un complexe plus stable avec l'antigène immobilisé que des anticorps dans ladite seconde sous-population, et dans laquelle la méthode comprend l'ajustement de données de détection de phase de dissociation à un modèle cinétique qui décrit la dissociation simultanée à partir de deux sites monovalents indépendants, un avec une phase de dissociation plus rapide, et un avec une phase de dissociation plus lente.

2. La méthode selon la revendication 1, dans laquelle le modèle cinétique est une équation à deux composantes :

$$R = R_1 * e^{-k_{d1}(t-t_0)} + R_2 * e^{-k_{d2}(t-t_0)}$$

où R est le temps de réponse de détection à l'instant t, $R_1$ et $R_2$ sont les contributions de réponse en provenance des sites respectivement avec les phases de dissociation plus rapide et plus lente, au commencement de la dissociation à l'instant $t_0$, et $k_{d1}$ et $k_{d2}$ sont les constantes de vitesse de dissociation pour les sites respectivement avec les phases de dissociation plus rapide et plus lente.

3. La méthode selon la revendication 2, dans laquelle les constantes de vitesse de dissociation déterminées par l'ajustement sont transformées en demi-vies ($t_{1/2}$) par l'équation :

$$t_{1/2} = \frac{\ln 2}{k_d}$$

où $t_{1/2}$ est le temps exigé pour que la quantité de complexe anticorps-antigène soit réduite à 50 % pendant la dissociation.

4. La méthode selon la revendication 3, dans laquelle les résultats de l'ajustement sont présentés par quatre paramètres : fraction rapide, $t_{1/2}$ rapide, fraction lente, $t_{1/2}$ lente.

5. La méthode selon l'une quelconque des revendications 1 à 4, dans laquelle l'antigène est un médicament.

6. La méthode selon l'une quelconque des revendications 1 à 4, dans laquelle l'antigène est un vaccin.

7. Une méthode de détermination de l'immunogénicité d'un médicament, qui comprend le contrôle de l'apparition d'anticorps anti-médicament chez un patient au cours du temps par la méthode selon l'une quelconque des revendications 1 à 4, et de détermination de n'importe quel décalage de proportions entre des anticorps plus stables et moins stables.

PRIOR ART

**FIG. 1**

PRIOR ART

FIG. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 08033073 A1
- US 2007016378 A1
- US 5313264 A
- US 5242828 A
- US 5436161 A
- US 5492840 A

**Non-patent literature cited in the description**

- **STECKBECK J.D. et al.** *J. Med. Primatolog.,* 2006, vol. 35, 248-260
- **BRUDERER U. et al.** *Journal of Immunological Methods,* July 1992, vol. 151 (1-2), 157-164
- **RILEY et al.** *Journal of Immunology,* 1980, vol. 124 (1), 1-7
- **HERRON J.N. et al.** Molecular Immunology. Pergamon, December 1993, vol. 20, 1323-1332
- **SEM D. S. et al.** Archives of Biochemistry and Biophysics. Academic press, December 1999, vol. 373, 62-68
- **KRUSE V.** Scandinavian Journal of Clinical and Laboratory Investigation. Informa Healthcare, May 1979, vol. 39, 215-221
- **YUE D.K. et al.** *Biochimica et Biophysica Acta- General subjects,* August 1976, vol. 444, 231-239